# EUROPEAN PATENT APPLICATION

(11) **EP 3 610 877 A1**
(43) Date of publication of application: **19.02.2020**
(21) Application number: 19199776.6
(22) Date of filing: 18.09.2017
(51) Int. Cl.: A61K 31/573, A61K 31/167, A61K 9/12, A61K 47/10, A61K 47/24, A61P 11/00

(54) **PHARMACEUTICAL COMPOSITION**

(30) Priority: 19.09.2016 GB 201615908; 02.12.2016 GB 201620515
(62) Divisional of application: 17777636.6
(71) Applicant: Mexichem Fluor S.A. de C.V., San Luis Potosi, S.L.P. C.P. 78395 (MX)
(72) Inventor: CORR, Stuart, Warrington, Cheshire WA4 5DH (GB); NOAKES, Timothy James, Nr Mold, Flintshire CH7 5JF (GB)
(74) Representative: Potter Clarkson

(57) **Abstract**

A method of improving the stability of a pharmaceutical composition comprising a propellant component, a drug component comprising at least one beclomethasone compound selected from beclomethasone and beclomethasone dipropionate, and ethanol is described. The method comprises using a propellant component comprising 1,1-difluoroethane (HFA-152a).

## Description

The present invention relates to the delivery of drug formulations from a medical device, such as a metered dose inhaler (MDI), using a propellant comprising 1,1-difluoroethane (HFA-152a). More particularly, the present invention relates to pharmaceutical compositions comprising HFA-152a propellant and a drug formulation which is dissolved or suspended in the propellant and to medical devices containing those compositions. The pharmaceutical compositions of the invention are particularly suited for delivery from a pressurised aerosol container using a metered dose inhaler (MDI).

MDIs are the most significant type of inhalation drug delivery system and are well known to those skilled in the art. They are designed to deliver, on demand, a discrete and accurate amount of a drug to the respiratory tract of a patient using a liquefied propellant in which the drug is dissolved, suspended or dispersed. The design and operation of MDIs is described in many standard textbooks and in the patent literature. They all comprise a pressurised container that holds the drug formulation, a nozzle and a valve assembly that is capable of dispensing a controlled quantity of the drug through the nozzle when it is activated. The nozzle and valve assembly are typically located in a housing that is equipped with a mouth piece. The drug formulation will comprise a propellant, in which the drug is dissolved, suspended or dispersed, and may contain other materials such as polar excipients, surfactants and preservatives.

In order for a propellant to function satisfactorily in MDIs, it needs to have a number of properties. These include an appropriate boiling point and vapour pressure so that it can be liquefied in a closed container at room temperature but develop a high enough pressure when the MDI is activated to deliver the drug as an atomised formulation even at low ambient temperatures. Further, the propellant should be of low acute and chronic toxicity and have a high cardiac sensitisation threshold. It should have a high degree of chemical stability in contact with the drug, the container and the metallic and non-metallic components of the MDI device, and have a low propensity to extract low molecular weight substances from any elastomeric materials in the MDI device. The propellant should also be capable of maintaining the drug in a homogeneous solution, in a stable suspension or in a stable dispersion for a sufficient time to permit reproducible delivery of the drug in use. When the drug is in suspension in the propellant, the density of the liquid propellant is desirably similar to that of the solid drug in order to avoid rapid sinking or floating of the drug particles in the liquid. Finally, the propellant should not present a significant flammability risk to the patient in use. In particular, it should form a non-flammable or low flammability mixture when mixed with air in the respiratory tract.

Dichlorodifluoromethane (R-12) possesses a suitable combination of properties and was for many years the most widely used MDI propellant, often blended with trichlorofluoromethane (R-11). Due to international concern that fully and partially halogenated chlorofluorocarbons (CFCs), such as dichlorodifluoromethane and trichlorofluoromethane, were damaging the earth's protective ozone layer, many countries entered into an agreement, the Montreal Protocol, stipulating that their manufacture and use should be severely restricted and eventually phased out completely. Dichlorodifluoromethane and trichlorofluoromethane were phased out for refrigeration use in the 1990's, but are still used in small quantities in the MDI sector as a result of an essential use exemption in the Montreal Protocol.

1,1,1,2-tetrafluoroethane (HFA-134a) was introduced as a replacement refrigerant and MDI propellant for R-12. 1,1,1,2,3,3,3-heptafluoropropane (HFA-227ea) was also introduced as a replacement propellant for dichlorotetrafluoroethane (R-114) in the MDI sector and is sometimes used alone or blended with HFA-134a for this application.

Although HFA-134a and HFA-227ea have low ozone depletion potentials (ODPs), they have global warming potentials (GWPs), 1430 and 3220 respectively, which are now considered to be too high by some regulatory bodies, especially for dispersive uses when they are released into the atmosphere.

One industrial area that has received particular attention recently has been the automotive air-conditioning sector where the use of HFA-134a has come under regulatory control as a result of the European Mobile Air Conditioning Directive (2006/40/EC). Industry is developing a number of possible alternatives to HFA-134a in automotive air conditioning and other applications that have a low greenhouse warming potential (GWP) as well as a low ozone depletion potential (ODP). Many of these alternatives include hydrofluoropropenes, especially the tetrafluoropropenes, such as 2,3,3,3-tetrafluoropropene (HFO-1234yf) and 1,3,3,3-tetrafluoropropene (HFO-1234ze).

Although the proposed alternatives to HFA-134a have a low GWP, the toxicological status of many of the components, such as certain of the fluoropropenes, is unclear and they are unlikely to be acceptable for use in the MDI sector for many years, if at all.

Beclomethasone and beclomethasone dipropionate (BDP) are corticosteroids that are used extensively as anti-inflammatory agents in the treatment of many respiratory tract and related disorders, including particularly asthma and chronic obstructive pulmonary disease (COPD). Both drugs are conveniently delivered using a MDI. BDP has also found use in combination therapies with long acting, beta-2-agonists (LABAs), including formoterol and formoterol fumarate dihydrate (FFD), in the treatment and control of asthma and COPD.

It is known that both BDP and FFD are relatively labile species that undergo significant degradation when formulated for delivery using a MDI, often resulting in a significant reduction in the storage life of formulated products. Whilst this reduced stability can be partly ameliorated through refrigerated storage, this is not always possible or convenient. Other approaches to improving the stability of FFD/BDP MDI formulations include the incorporation of a mineral acid such as HCl or an organic acid such as citric acid resulting in relatively acidic formulations. However, such acidic formulations have the potential for incompatibility with the materials of construction of a MDI device including the potential to corrode the aluminium cans in which the formulations are typically contained. Whilst this corrosion problem can be addressed through coating aluminum cans with inert polymeric coatings or through the use of stainless steel cans, both approaches add additional cost and/or complexity to the manufacture of MDIs.

There is a need for a pharmaceutical composition comprising a beclomethasone compound, such as beclomethasone dipropionate (BDP), and a long acting, beta-2-agonist (LABAs), such as formoterol fumarate dihydrate, which can be delivered using a MDI and that uses a propellant having a reduced GWP in comparison with HFA-134a and HFA-227ea. There is also a need for a pharmaceutical composition which exhibits satisfactory stability without the use of acid stabilizers.

We have found that the issues associated with the use of beclomethasone-based formulations in MDIs may be overcome by using a propellant that comprises 1,1-difluoroethane (HFA-152a), particularly where the formulations contain low amounts of water. These formulations can exhibit improved chemical stability, improved aerosolisation performance for improved drug delivery, good suspension stability, reduced GWP, good compatibility with standard uncoated aluminium cans as well as good compatibility with standard valves and seals.

According to a first aspect of the present invention, there is provided a pharmaceutical composition, e.g. a pharmaceutical suspension or a pharmaceutical solution, said composition comprising:
(i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate;
(ii) a propellant component comprising 1,1-difluoroethane (HFA-152a); and
(iii) glycerol.

The pharmaceutical composition of the first aspect of the invention typically contains less than 500 ppm of water based on the total weight of the pharmaceutical composition. The improved chemical stability is observed, in particular, when the pharmaceutical composition contains less than 100 ppm, preferably less than 50 ppm, more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition of the first aspect may contain greater than 0.5 ppm of water, e.g. greater than 1 ppm, but less than the amounts discussed above, as it can in practice be difficult to remove all the water from the composition and then retain it in such a water-free state.

Accordingly a preferred embodiment of the first aspect of the present invention provides a pharmaceutical composition, e.g. a pharmaceutical suspension or a pharmaceutical solution, said composition comprising:
(i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate;
(ii) a propellant component comprising 1,1-difluoroethane (HFA-152a); and
(iii) glycerol,
wherein the composition contains less than 100 ppm, preferably less than 50 ppm, more preferably less than 10 ppm and especially less than 5 ppm of water based on the total weight of the pharmaceutical composition.

In a preferred embodiment, the pharmaceutical composition of the first aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the first aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Accordingly a preferred embodiment of the first aspect of the present invention provides a pharmaceutical composition, e.g. a pharmaceutical suspension or a pharmaceutical solution, said composition comprising:
(i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate;
(ii) a propellant component comprising 1,1-difluoroethane (HFA-152a); and
(iii) glycerol,
wherein the composition contains less than 1000 ppm, preferably less than 500 ppm, more preferably less than 100 ppm and especially less than 50 ppm of oxygen based on the total weight of the pharmaceutical composition.

The pharmaceutical composition of the present invention is suitable for delivery to the respiratory tract using a metered dose inhaler (MDI).

The at least one beclomethasone compound and the at least one long acting beta-2-agonist in the pharmaceutical composition of the invention in all aspects and embodiments disclosed herein are preferably in a micronized form. Further, the pharmaceutical composition of the invention in all aspects and embodiments disclosed herein is preferably free of perforated microstructures.

The pharmaceutical composition of the first aspect of the invention includes a drug component comprising at least one beclomethasone compound selected from beclomethasone and the pharmaceutically acceptable derivatives thereof. A particularly preferred beclomethasone compound is beclomethasone dipropionate (BDP).

The drug component also includes at least one long acting beta-2-agonist (LABA). Any of the long acting beta-2-agonists that have been in use hitherto for treating asthma and chronic obstructive pulmonary diseases and that can be delivered using a MDI can be used in the pharmaceutical compositions of the present invention. Suitable long acting beta-2-agonists include formoterol, arformoterol, bambuterol, clenbuterol, salmeterol, indacaterol, olodaterol and vilanterol as well as their pharmaceutically acceptable derivatives, such as their pharmaceutically acceptable salts.

Preferred long acting beta-2-agonists are selected from formoterol, the pharmaceutically acceptable salts of formoterol, the hydrates of formoterol and the hydrates of pharmaceutically acceptable salts of formoterol. Suitable pharmaceutically acceptable salts of formoterol include acid addition salts derived from organic and inorganic acids, such as the hydrochloride, sulphate, phosphate, maleate, fumarate, tartrate, citrate, benzoate, methoxybenzoate, hydroxybenzoate, chlorobenzoate, p-toluenesulphonate, methanesulphonate, ascorbate, salicylate, acetate, succinate, lactate, glutarate, gluconate and oleate.

The fumarate salt of formoterol is preferred and in a particularly preferred embodiment the pharmaceutical composition of the invention includes formoterol fumarate dihydrate. Especially preferred pharmaceutical compositions of the invention are those in which the at least one long acting beta-2-agonist consists essentially of formoterol fumarate dihydrate. By the term "consists essentially of", we mean that at least 95 weight %, more preferably at least 98 weight % and especially at least 99 weight % of the at least one long acting beta-2-agonist is formoterol fumarate dihydrate. Most preferred are pharmaceutical compositions in which the at least one long acting beta-2-agonist is entirely formoterol fumarate dihydrate.

The at least one beclomethasone compound and/or the at least one long acting beta-2-agonist may be dispersed or suspended in the propellant. The drug particles in such suspensions preferably have a diameter of less than 100 microns, e.g. less than 50 microns. However, in an alternative embodiment the pharmaceutical compositions of the invention are solutions with the at least one beclomethasone compound (BDP) and the at least one long acting beta-2-agonist dissolved in the propellant, e.g. with the assistance of a polar excipient, such as ethanol.

The amount of the drug component in the pharmaceutical composition of the first aspect of the present invention will typically be in the range of from 0.01 to 2.5 weight % based on the total weight of the pharmaceutical composition. Preferably, the drug component will comprise from 0.01 to 2.0 weight %, more preferably from 0.05 to 2.0 weight % and especially from 0.05 to 1.5 weight % of the total weight of the pharmaceutical composition. The drug component may consist essentially of or consist entirely of the at least one beclomethasone compound, especially beclomethasone dipropionate, and the at least one long acting beta-2-agonist, especially formoterol fumarate dihydrate. By the term "consists essentially of", we mean that at least 98 weight %, more preferably at least 99 weight % and especially at least 99.9 weight % of the drug component consists of the at least one beclomethasone compound and the at least one long acting beta-2-agonist. Alternatively, the drug component may contain other drugs, such as at least one long acting muscarinic antagonist (LAMA).

In one preferred embodiment, the pharmaceutical composition and more specifically the drug component thereof is free of pharmaceutically acceptable salts of both cromoglycic acid and nedocromil.

The propellant component in the pharmaceutical composition of the first aspect of the present invention comprises 1,1-difluoroethane (HFA-152a). Thus, we do not exclude the possibility that the propellant component may include other propellant compounds in addition to the HFA-152a. For example, the propellant component may additionally comprise one or more additional hydrofluorocarbon or hydrocarbon propellant compounds, e.g. selected from HFA-227ea, HFA-134a, difluoromethane (HFA-32), propane, butane, isobutane and dimethyl ether. The preferred additional propellants are HFA-227ea and HFA-134a.

If an additional propellant compound is included, such as HFA-134a or HFA-227ea, at least 5 % by weight, preferably at least 10 % by weight and more preferably at least 50 % by weight of the propellant component should be HFA-152a. Typically, the HFA-152a will constitute at least 90 weight %, e.g. from 90 to 99 weight %, of the propellant component. Preferably, the HFA-152a will constitute at least 95 weight %, e.g. from 95 to 99 weight %, and more preferably at least 99 weight % of the propellant component.

In a preferred embodiment, the propellant component has a global warming potential (GWP) of less than 250, more preferably less than 200 and still more preferably less than 150.

In an especially preferred embodiment, the propellant component consists entirely of HFA-152a so that the pharmaceutical composition of the invention comprises HFA-152a as the sole propellant. By the term "consists entirely of" we do not, of course, exclude the presence of minor amounts, e.g. up to a few hundred parts per million, of impurities that may be present following the process that is used to make the HFA-152a providing that they do not affect the suitability of the propellant in medical applications. Preferably the HFA-152a propellant will contain no more than 10 ppm, e.g. from 0.5 to 10 ppm, more preferably no more than 5 ppm, e.g. from 1 to 5 ppm, of unsaturated impurities, such as vinyl fluoride, vinyl chloride, vinylidene fluoride and chloro-fluoro ethylene compounds.

The amount of propellant component in the pharmaceutical composition of the invention will vary depending on the amounts of the drugs and other components in the pharmaceutical composition. Typically, the propellant component will comprise from 80.0 to 99.99 weight % of the total weight of the pharmaceutical composition. Preferably, the propellant component will comprise from 90.0 to 99.99 weight %, more preferably from 96.5 to 99.99 weight % and especially from 97.5 to 99.95 weight % of the total weight of the pharmaceutical composition.

The amount of glycerol in the pharmaceutical composition of the first aspect of the present invention will typically be in the range of from 0.05 to 5.0 weight % based on the total weight of the pharmaceutical composition. Preferably, the glycerol will comprise from 0.1 to 3.0 weight %, more preferably from 0.1 to 2.5 weight % and especially from 0.5 to 2.5 weight % of the total weight of the pharmaceutical composition.

In one embodiment, the pharmaceutical composition of the first aspect of the present invention consists essentially of and more preferably consists entirely of the three components (i) to (iii) listed above. By the term "consists essentially of", we mean that at least 98 weight %, more preferably at least 99 weight % and especially at least 99.9 weight % of the pharmaceutical composition consists of the three listed components.

In another embodiment, the pharmaceutical composition of the first aspect of the present invention additionally includes a polar excipient, such as ethanol. Polar excipients have been used previously in pharmaceutical compositions for treating respiratory disorders that are delivered using metered dose inhalers (MDIs). They are also referred to as solvents, co-solvents, carrier solvents and adjuvants. Their inclusion can serve to solubilise the surfactant or the drug in the propellant and/or inhibit deposition of drug particles on the surfaces of the metered dose inhaler that are contacted by the pharmaceutical composition as it passes from the container in which it is stored to the nozzle outlet. They are also used as bulking agents in two-stage filling processes where the drug is mixed with a suitable polar excipient. The most commonly used polar excipient is ethanol. If a polar excipient is used, it will typically be present in an amount of from 0.5 to 15 % by weight, preferably in an amount of from 0.5 to 10 % by weight, and more preferably in an amount of from 1 to 5 % by weight based on the total weight of the pharmaceutical composition.

Even those pharmaceutical compositions of the invention that contain further components in addition to the defined drug component, the defined propellant component and the glycerol, such as a polar excipient, should be surfactant-free.

Accordingly a preferred embodiment of the first aspect of the present invention provides a pharmaceutical composition, e.g. a pharmaceutical suspension or a pharmaceutical solution, said composition comprising:
(i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate;
(ii) a propellant component comprising 1,1-difluoroethane (HFA-152a); and
(iii) glycerol,
wherein the composition is surfactant-free and preferably contains less than 100 ppm, more preferably less than 50 ppm, particularly less than 10 ppm and especially less than 5 ppm of water based on the total weight of the pharmaceutical composition.

In a preferred embodiment, the pharmaceutical composition of the first aspect of the present invention is free of acid stabilisers, such as organic and inorganic acids.

The pharmaceutical composition of the invention may also include a long acting muscarinic antagonist (LAMA). Any of the long acting muscarinic antagonists that have been in use hitherto for treating chronic obstructive pulmonary diseases and that can be delivered using a MDI can be used in the pharmaceutical compositions of the present invention. Suitable long acting muscarinic antagonists include umeclidinium, ipratropium, tiotropium, aclidinium and the pharmaceutically acceptable derivatives thereof, especially the pharmaceutically acceptable salts thereof. Preferred compounds include the pharmaceutically acceptable salts of glycopyrrolate (also known as glycopyrronium). Glycopyrrolate is a quaternary ammonium salt. Suitable pharmaceutically acceptable counter ions include, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1- hydroxynaphthalene-2-carboxylate, 3-hydroxynaphthalene-2-carboxylate, methanesulfonate and benzenesulfonate. A preferred compound is the bromide salt of glycopyrrolate also known as glycopyrronium bromide.

According to a second aspect of the present invention, there is provided a pharmaceutical composition, e.g. a pharmaceutical suspension or a pharmaceutical solution, said composition comprising:
(i) a drug component comprising at least one beclomethasone compound selected from beclomethasone and the pharmaceutically acceptable derivatives thereof, particularly beclomethasone dipropionate (BDP), at least one long acting beta-2-agonist, particularly formoterol fumarate dihydrate, and at least one long acting muscarinic antagonist, particularly at least one pharmaceutically acceptable salt of glycopyrrolate;
(ii) a propellant component comprising 1,1-difluoroethane (HFA-152a); and
(iii) glycerol.

The pharmaceutical composition of the second aspect of the invention typically contains less than 500 ppm of water based on the total weight of the pharmaceutical composition. Preferably, the pharmaceutical composition of the second aspect of the present invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the second aspect of the present invention is water-free. Alternatively, the pharmaceutical composition of the second aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the second aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the second aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Suitable and preferred long acting beta-2-agonists are as discussed above for the pharmaceutical composition of the first aspect of the present invention.

Typical and preferred amounts of the drug component and the propellant component in the pharmaceutical composition of the second aspect of the present invention and suitable, typical and preferred compositions for the propellant component are as discussed above for the pharmaceutical composition of the first aspect of the invention. The drug component may consist essentially of or consist entirely of the at least one beclomethasone compound, the at least one long acting beta-2-agonist, and the at least one long acting muscarinic antagonist. By the term "consists essentially of", we mean that at least 98 weight %, more preferably at least 99 weight % and especially at least 99.9 weight % of the drug component consists of the at least one beclomethasone compound, the at least one long acting beta-2-agonist, and the at least one long acting muscarinic antagonist.

In one embodiment, the pharmaceutical composition of the second aspect of the present invention consists essentially of and more preferably consists entirely of the three components (i) to (iii) listed above. By the term "consists essentially of", we mean that at least 98 weight %, more preferably at least 99 weight % and especially at least 99.9 weight % of the pharmaceutical composition consists of the three listed components.

In another embodiment, the pharmaceutical composition of the second aspect of the invention may contain a polar excipient as discussed above for the pharmaceutical composition of the first aspect of the invention. Suitable and preferred polar excipients are as discussed above for the pharmaceutical composition of the first aspect of the invention. Typical and preferred amounts of the polar excipient are as discussed above for the pharmaceutical composition of the first aspect of the invention.

In an especially preferred embodiment of the second aspect of the invention, the drug component comprises beclomethasone dipropionate, formoterol fumarate dihydrate and at least one pharmaceutically acceptable glycopyrrolate salt, especially glycopyrronium bromide. Preferably, the beclomethasone dipropionate, formoterol fumarate dihydrate and the at least one pharmaceutically acceptable glycopyrrolate salt are the only pharmaceutical actives in the pharmaceutical composition of the second aspect of the invention.

As with the pharmaceutical composition of the first aspect of the invention, the pharmaceutical composition of the second aspect of the invention should be surfactant-free. Furthermore, in a preferred embodiment, the pharmaceutical composition of the second aspect of the invention is free of acid stabilisers, such as organic and inorganic acids.

It has been found that the use of propellants comprising 1,1-difluoroethane (HFA-152a) in pharmaceutical compositions containing at least one beclomethasone compound selected from beclomethasone and the pharmaceutically acceptable derivatives thereof, glycerol, ethanol and the propellant can unexpectedly improve the chemical stability of the beclomethasone compound compared to the stability it exhibits in formulations containing either HFA-134a or HFA-227ea as the propellant.

Accordingly, in a third aspect of the present invention there is provided a method of improving the stability of a pharmaceutical composition comprising a propellant component, a drug component comprising at least one beclomethasone compound selected from beclomethasone and the pharmaceutically acceptable derivatives thereof, particularly beclomethasone dipropionate (BDP), glycerol and ethanol, said method comprising using a propellant component comprising 1,1-difluoroethane (HFA-152a).

The at least one beclomethasone compound may be dissolved or suspended in the pharmaceutical composition.

The improved chemical stability can result, in particular, when the pharmaceutical composition contains less than 500 ppm, preferably less than 100 ppm, more preferably less than 50 ppm, still more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition recited in the third aspect of the present invention may contain greater than 0.5 ppm of water, e.g. greater than 1 ppm, but less than the amounts discussed above, as it can in practice be difficult to remove all the water from the composition and then retain it in such a water-free state.

Accordingly, in a preferred embodiment of the third aspect of the present invention there is provided a method of improving the stability of a pharmaceutical composition comprising a propellant component, a drug component comprising at least one beclomethasone compound selected from beclomethasone and the pharmaceutically acceptable derivatives thereof, particularly beclomethasone dipropionate (BDP), glycerol and ethanol, said method comprising using a propellant component comprising 1,1-difluoroethane (HFA-152a) and selecting the components and conditions for the preparation of the pharmaceutical composition to maintain the water content of the pharmaceutical composition below 100 ppm, preferably below 50 ppm, more preferably below 10 ppm and particularly below 5 ppm based on the total weight of the pharmaceutical composition.

In practice, preparing a pharmaceutical composition with the low water levels recited above involves using a propellant component with a suitably low water content, as it is usually the largest mass item in the finished device, and then preparing the pharmaceutical composition under suitably dry conditions, e.g. in a dry nitrogen atmosphere. Preparing pharmaceutical compositions under dry conditions is well known and the techniques involved are well understood by those skilled in the art. Other steps to obtain a low water content in the finished device include drying and storing the can and valve components in a moisture-controlled atmosphere, e.g. dry nitrogen or air, prior to and during device assembly. If the pharmaceutical composition contains a significant amount of ethanol, then it may also be important to control the water content of the ethanol as well as the propellant, e.g. by drying to reduce the water content to suitably low levels. Suitable drying techniques are well known to those skilled in the art and include the use of a molecular sieve or other inorganic desiccant and membrane drying processes.

In the stabilisation method of the third aspect of the present invention suitable and preferred beclomethasone compounds are as described above for the pharmaceutical composition of the first aspect of the present invention. In addition, typical and preferred amounts of the drug component, the propellant component, the glycerol and the ethanol in the stabilisation method of the third aspect of the present invention and suitable, typical and preferred compositions for the propellant component are as discussed above for the pharmaceutical composition of the first aspect of the invention.

The drug component in the stabilisation method of the third aspect of the present invention may consist essentially of or consist entirely of the at least one beclomethasone compound. By the term "consists essentially of", we mean that at least 98 weight %, more preferably at least 99 weight % and especially at least 99.9 weight % of the drug component consists of the least one beclomethasone compound. Alternatively, the drug component may additionally comprise at least one long acting beta-2-agonist or at least one long acting beta-2-agonist together with at least one long acting muscarinic antagonist as discussed for the pharmaceutical compositions of the first and second aspects of the present invention. When a long acting beta-2-agonist either alone or together with a long acting muscarinic antagonist is included, suitable and preferred long acting beta-2-agonists and suitable and preferred long acting muscarinic antagonists are as described above for the pharmaceutical compositions of the first and second aspects of the present invention.

In one embodiment, the pharmaceutical composition in the stabilisation method of the third aspect of the present invention consists essentially of and more preferably consists entirely of the drug component, the propellant component, the glycerol and the ethanol as defined above. By the term "consists essentially of", we mean that at least 98 weight %, more preferably at least 99 weight % and especially at least 99.9 weight % of the pharmaceutical composition consists of the four components.

In a preferred embodiment, the pharmaceutical composition that is provided in the stabilisation method of the third aspect of the present invention is free of surfactants. In a particularly preferred embodiment, the pharmaceutical composition that is provided in the stabilisation method of the third aspect of the present invention is free of acid stabilisers, such as organic and inorganic acids.

In one preferred stabilisation method of the third aspect of the present invention, a pharmaceutical composition containing up to 15 weight % of ethanol based on the total weight of the pharmaceutical composition will produce less than 2.0 % by weight, preferably less than 1.5 % by weight and more preferably less than 1.0 % by weight of impurities from the degradation of the at least one beclomethasone compound based on the total weight of the at least one beclomethasone compound and the impurities after storage at 40°C and 75 % relative humidity for 1 month.

In another preferred stabilisation method of the third aspect of the present invention, a pharmaceutical composition containing up to 15 weight % of ethanol based on the total weight of the pharmaceutical composition will produce less than 2.5 % by weight, preferably less than 2.0 % by weight and more preferably less than 1.5 % by weight of impurities from the degradation of the at least one beclomethasone compound based on the total weight of the at least one beclomethasone compound and the impurities after storage at 40°C and 75 % relative humidity for 3 months.

One preferred pharmaceutical composition of the first and second aspects of the present invention containing up to 15 weight % of ethanol based on the total weight of the pharmaceutical composition will produce less than 2.0 % by weight, preferably less than 1.5 % by weight and more preferably less than 1.0 % by weight of total impurities from the degradation of the at least one beclomethasone compound after storage at 40°C and 75 % relative humidity for 1 month.

Another preferred pharmaceutical composition of the first and second aspects of the present invention containing up to 15 weight % of ethanol based on the total weight of the pharmaceutical composition will produce less than 2.5 % by weight, preferably less than 2.0 % by weight and more preferably less than 1.5 % by weight of total impurities from the degradation of the at least one beclomethasone compound after storage at 40°C and 75 % relative humidity for 3 months.

The weight % of impurities indicated above are based on the total weight of the at least one beclomethasone compound and the impurities.

In referring to the storage of the pharmaceutical compositions in the above described stabilisation methods, we are referring, in particular, to the storage of those compositions in uncoated aluminium containers. Similarly, in referring to the storage of the above described pharmaceutical compositions, we are referring, in particular, to their storage in uncoated aluminium containers.

The pharmaceutical compositions of the invention find particular utility in the delivery of the drug component from a pressurised aerosol container, e.g. using a metered dose inhaler (MDI). For this application, the pharmaceutical compositions are contained in the pressurised aerosol container and the HFA-152a propellant functions to deliver the drug component as a fine aerosol spray.

The pharmaceutical compositions of the invention may comprise one or more other additives of the type that are conventionally used in drug formulations for pressurised MDIs, such as valve lubricants. Where other additives are included in the pharmaceutical compositions, they are normally used in amounts that are conventional in the art.

The pharmaceutical compositions of the invention are normally stored in a pressurised container or canister which is to be used in association with a medication delivery device. When so stored, the pharmaceutical compositions are normally a liquid. In a preferred embodiment, the pressurised container is designed for use in a metered dose inhaler (MDI). In a particularly preferred embodiment, the pressurised container is a coated aluminium can or an uncoated aluminium can, especially the latter.

Accordingly, a fourth aspect of the present invention provides a pressurised container holding the pharmaceutical composition of the first or second aspect of the present invention. In a fifth aspect, the present invention provides a medication delivery device, especially a metered dose inhaler, having a pressurised container holding the pharmaceutical composition of the first or second aspect of the present invention.

The metered dose inhaler typically comprises a nozzle and valve assembly that is crimped to a container holding the pharmaceutical composition to be dispensed. An elastomeric gasket is used to provide a seal between the container and the nozzle/valve assembly. Preferred elastomeric gasket materials are EPDM, chlorobutyl, bromobutyl and cycloolefin copolymer rubbers as these can exhibit good compatibility with HFA-152a and also provide a good barrier to prevent or limit HFA-152a permeating from the container.

The pharmaceutical compositions of the present invention are for use in medicine for treating a patient suffering or likely to suffer from a respiratory disorder and especially asthma or a chronic obstructive pulmonary disease.

Accordingly, the present invention also provides a method for treating a patient suffering or likely to suffer from a respiratory disorder, especially asthma or a chronic obstructive pulmonary disease, which comprises administering to the patient a therapeutically or prophylactically effective amount of a pharmaceutical composition as discussed above. The pharmaceutical composition is preferably delivered to the patient using a MDI.

The pharmaceutical compositions of the invention can be prepared and the MDI devices filled using techniques that are standard in the art, such as pressure filling and cold filling. For example, the pharmaceutical compositions can be prepared by a simple blending operation in which the at least one beclomethasone compound, the at least one long acting beta-2 agonist, the glycerol, optionally the at least one long acting muscarinic antagonist, optionally the polar excipient, and the HFA-152a-containing propellant are mixed together in the required proportions in a suitable mixing vessel. Mixing can be promoted by stirring as is common in the art. Conveniently, the HFA-152a-containing propellant is liquefied to aid mixing. If the pharmaceutical composition is made in a separate mixing vessel, it can then be transferred to pressurised containers for storage, such as pressurised containers that are used as part of medication delivery devices and especially MDIs.

The pharmaceutical compositions of the invention can also be prepared within the confines of a pressurised container, such as an aerosol canister or vial, from which the compositions are ultimately released as an aerosol spray using a medication delivery device, such as a MDI. In this method, a weighed amount of the at least one beclomethasone compound, the at least one long acting beta-2 agonist, the glycerol, optionally the at least one long acting muscarinic antagonist and optionally the polar excipient are introduced into the open container. A valve is then crimped onto the container and the HFA-152a-containing propellant component, in liquid form, introduced through the valve into the container under pressure, optionally after first evacuating the container through the valve. Other components, if included, can be mixed with the drug component or, alternatively, introduced into the container after the valve has been fitted, either alone or as a premix with the propellant component. The whole mixture can then be treated to disperse the drugs in the propellant component, e.g. by vigorous shaking or using an ultrasonic bath. Suitable containers may be made of plastics, metal, e.g. aluminium, or glass. Preferred containers are made of metal, especially aluminium which may be coated or uncoated. Uncoated aluminium containers are especially preferred.

The container may be filled with enough of the pharmaceutical composition to provide for a plurality of dosages. The pressurized aerosol canisters that are used in MDIs typically contain 50 to 150 individual dosages.

The present invention also provides a method of reducing the global warming potential (GWP) of a pharmaceutical composition comprising: (i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate; (ii) a propellant component; and (iii) glycerol, said method comprising using a propellant component comprising 1,1-difluoroethane (HFA-152a). This method is applicable to the preparation of all the pharmaceutical compositions disclosed herein in all their aspects and embodiments.

Preferably, at least 90 weight %, more preferably at least 95 weight % and still more preferably at least 99 weight % of the propellant component used is HFA-152a. In an especially preferred embodiment, the propellant component used is entirely HFA-152a.

The propellant component that is used will preferably have a global warming potential (GWP) of less than 250, more preferably less than 200 and still more preferably less than 150.

Additional aspects and embodiments of the present invention are described in the following numbered paragraphs.
[1] A pharmaceutical composition comprising:
   (i) a drug component comprising at least one beclomethasone compound selected from beclomethasone and the pharmaceutically acceptable derivatives thereof and at least one long acting beta-2-agonist;
   (ii) a propellant component comprising 1,1-difluoroethane (HFA-152a); and
   (iii) glycerol.
[2] The pharmaceutical composition of paragraph [1], wherein the composition contains less than 500 ppm, preferably less than 100 ppm, more preferably less than 50 ppm, still more preferably less than 10 ppm and especially less than 5 ppm of water based on the total weight of the pharmaceutical composition.
[3] The pharmaceutical composition of paragraph [2], wherein the composition contains greater than 0.5 ppm, e.g. greater than 1 ppm, of water based on the total weight of the pharmaceutical composition.
[4] The pharmaceutical composition of any one of paragraphs [1] to [3], wherein the composition contains less than 1000 ppm, preferably less than 500 ppm, more preferably less than 100 ppm and particularly less than 50 ppm of oxygen based on the total weight of the pharmaceutical composition.
[5] The pharmaceutical composition of paragraph [4], wherein the composition contains greater than 0.5 ppm, e.g. greater than 1 ppm, of oxygen based on the total weight of the pharmaceutical composition.
[6] The pharmaceutical composition of any one of paragraphs [1] to [5], wherein the at least one beclomethasone compound is beclomethasone dipropionate.
[7] The pharmaceutical composition of any one of paragraphs [1] to [6], wherein the at least one long acting beta-2-agonist (LABA) is selected from the group consisting of formoterol, arformoterol, bambuterol, clenbuterol, salmeterol, indacaterol, olodaterol, vilanterol and the pharmaceutically acceptable derivatives thereof.
[8] The pharmaceutical composition of paragraph [7], wherein the at least one long acting beta-2-agonist (LABA) is selected from the group consisting of formoterol, the pharmaceutically acceptable salts of formoterol, the hydrates of formoterol and the hydrates of pharmaceutically acceptable salts of formoterol.
[9] The pharmaceutical composition of paragraph [7], wherein the at least one long acting beta-2-agonist (LABA) is formoterol fumarate dihydrate.
[10] The pharmaceutical composition of any one of paragraphs [1] to [9], wherein the at least one beclomethasone compound and the at least one long acting beta-2-agonist are each in a micronized form.
[11] The pharmaceutical composition of any one of paragraphs [1] to [10], wherein the drug component additionally comprises at least one long acting muscarinic antagonist.
[12] The pharmaceutical composition of paragraph [11], wherein the at least one long acting muscarinic antagonist is selected from the group consisting of umeclidinium, ipratropium, tiotropium, aclidinium and the pharmaceutically acceptable derivatives thereof.
[13] The pharmaceutical composition of paragraph [11], wherein the at least one long acting muscarinic antagonist is a pharmaceutically acceptable salt of glycopyrrolate, especially glycopyrronium bromide.
[14] The pharmaceutical composition of any one of paragraphs [11] to [13], wherein the at least one long acting muscarinic antagonist is in a micronized form.
[15] The pharmaceutical composition of any one of paragraphs [1] to [14], wherein the drug component comprises from 0.01 to 2.5 weight %, preferably from 0.01 to 2.0 weight %, more preferably from 0.05 to 2.0 weight % and especially from 0.05 to 1.5 weight % of the total weight of the pharmaceutical composition.
[16] The pharmaceutical composition of any one of paragraphs [1] to [15], wherein the propellant component comprises from 80.0 to 99.99 weight %, preferably from 90.0 to 99.99 weight %, more preferably from 96.5 to 99.99 weight % and especially from 97.5 to 99.95 weight % of the total weight of the pharmaceutical composition.
[17] The pharmaceutical composition of any one of paragraphs [1] to [16], wherein at least 90 weight %, preferably at least 95 weight % and more preferably at least 99 weight % of the propellant component is 1,1-difluoroethane (HFA-152a).
[18] The pharmaceutical composition of any one of paragraphs [1] to [16], wherein the propellant component is entirely 1,1-difluoroethane (HFA-152a).
[19] The pharmaceutical composition of paragraph [17] or [18], wherein the propellant component contains from 0.5 to 10 ppm, e.g. from 1 to 5 ppm, of unsaturated impurities.
[20] The pharmaceutical composition of any one of paragraphs [1] to [19], wherein at least 95 weight %, preferably at least 98 weight % and more preferably at least 99 weight % of the composition consists of the three components (i), (ii) and (iii).
[21] The pharmaceutical composition of any one of paragraphs [1] to [20] further comprising a polar excipient.
[22] The pharmaceutical composition of paragraph [21], wherein the polar excipient is ethanol.
[23] The pharmaceutical composition of any one of paragraphs [1] to [19] which consists entirely of the three components (i), (ii) and (iii).
[24] The pharmaceutical composition of any one of paragraphs [1] to [22] which is surfactant-free.
[25] The pharmaceutical composition of any one of paragraphs [1] to [22] and [24] which is free of acid stabilisers.
[26] The pharmaceutical composition of any one of paragraphs [1] to [25] which produces less than 2.0 % by weight, preferably less than 1.5 % by weight and more preferably less than 1.0 % by weight of total impurities from the degradation of the at least one beclomethasone compound after storage in uncoated aluminium containers at 40°C and 75 % relative humidity for 1 month for amounts of ethanol up to 15 weight % based on the total weight of the pharmaceutical composition.
[27] The pharmaceutical composition of any one of paragraphs [1] to [26] which produces less than 2.5 % by weight, preferably less than 2.0 % by weight and more preferably less than 1.5 % by weight of total impurities from the degradation of the at least one beclomethasone compound after storage in uncoated aluminium containers at 40°C and 75 % relative humidity for 3 months for amounts of ethanol up to 15 weight % based on the total weight of the pharmaceutical composition.
[28] The pharmaceutical composition of any one of paragraphs [1] to [27] in the form of a suspension.
[29] The pharmaceutical composition of any one of paragraphs [1] to [27] in the form of a solution.
[30] The pharmaceutical composition of any one of paragraphs [1] to [29] which is free of perforated microstructures.
[31] The pharmaceutical composition of any one of paragraphs [1] to [30] which is free of pharmaceutically acceptable salts of both cromoglycic acid and nedocromil.
[32] The pharmaceutical composition of any one of paragraphs [1] to [31] which is free of polymers having amide and/or carboxylic acid ester repeating structural units.
[33] The pharmaceutical composition of any one of paragraphs [1] to [32], wherein the propellant component has a global warming potential (GWP) of less than 250, preferably less than 200 and more preferably less than 150.
[34] A sealed container that contains a pharmaceutical composition as paragraphed in any one of paragraphs [1] to [33].
[35] The sealed container of paragraph [34] which is an uncoated aluminium can.
[36] The sealed container of paragraph [34] or paragraph [35] which is a pressurized aerosol container for use with a metered dose inhaler (MDI).
[37] A metered dose inhaler (MDI) fitted with a sealed container as paragraphed in paragraph [36].
[38] The metered dose inhaler of paragraph [37] which comprises a nozzle and valve assembly attached to the pressurized aerosol container and a gasket made from an elastomeric material selected from EPDM, chlorobutyl, bromobutyl and cycloolefin copolymer rubbers to provide a seal between the container and the nozzle/valve assembly.
[39] A method for treating a patient suffering or likely to suffer from a respiratory disorder which comprises administering to the patient a therapeutically or prophylactically effective amount of a pharmaceutical composition as paragraphed in any one of paragraphs [1] to [33].
[40] The method of paragraph [39], wherein the respiratory disorder is asthma or a chronic obstructive pulmonary disease.
[41] The method of paragraph [39] or [40], wherein the pharmaceutical composition is delivered to the patient using a metered dose inhaler (MDI).
[42] A method of improving the stability of a pharmaceutical composition comprising a propellant component, a drug component comprising at least one beclomethasone compound selected from beclomethasone and the pharmaceutically acceptable derivatives thereof, glycerol and ethanol, said method comprising using a propellant component comprising 1,1-difluoroethane (HFA-152a).
[43] The method of paragraph [42], further comprising selecting the components and conditions for the preparation of the pharmaceutical composition to maintain the water content of the pharmaceutical composition below 500 ppm, preferably below 100 ppm, more preferably below 50 ppm, still more preferably below 10 ppm and particularly below 5 ppm based on the total weight of the pharmaceutical composition.
[44] The method of paragraph [42] or [43], wherein the oxygen content of the resulting pharmaceutical composition is below 1000 ppm, preferably below 500 ppm, more preferably below 100 ppm and particularly below 50 ppm based on the total weight of the pharmaceutical composition.
[45] The method of any one of paragraphs [42] to [44], wherein the at least one beclomethasone compound is beclomethasone dipropionate.
[46] The method of any one of paragraphs [42] to [45], wherein the at least one beclomethasone compound is in a micronized form.
[47] The method of any one of paragraphs [42] to [46], wherein the drug component additionally comprises at least one long acting beta-2-agonist (LABA).
[48] The method of paragraph [47], wherein the at least one long acting beta-2-agonist (LABA) is selected from the group consisting of formoterol, arformoterol, bambuterol, clenbuterol, salmeterol, indacaterol, olodaterol, vilanterol and the pharmaceutically acceptable derivatives thereof.
[49] The method of paragraph [47], wherein the at least one long acting beta-2-agonist (LABA) is selected from the group consisting of formoterol, the pharmaceutically acceptable salts of formoterol, the hydrates of formoterol and the hydrates of pharmaceutically acceptable salts of formoterol.
[50] The method of paragraph [47], wherein the at least one long acting beta-2-agonist (LABA) is formoterol fumarate dihydrate.
[51] The method of any one of paragraphs [47] to [50], wherein the at least one long acting beta-2-agonist is in a micronized form.
[52] The method of any one of paragraphs [42] to [51], wherein the drug component additionally comprises at least one long acting muscarinic antagonist.
[53] The method of paragraph [52], wherein the at least one long acting muscarinic antagonist is selected from the group consisting of umeclidinium, ipratropium, tiotropium, aclidinium and the pharmaceutically acceptable derivatives thereof.
[54] The method of paragraph [52], wherein the at least one long acting muscarinic antagonist is a pharmaceutically acceptable salt of glycopyrrolate, especially glycopyrronium bromide.
[55] The method of any one of paragraphs [52] to [54], wherein the at least one long acting muscarinic antagonist is in a micronized form.
[56] The method of any one of paragraphs [42] to [55], wherein the drug component comprises from 0.01 to 2.5 weight %, preferably from 0.01 to 2.0 weight %, more preferably from 0.05 to 2.0 weight % and especially from 0.05 to 1.5 weight % of the total weight of the pharmaceutical composition.
[57] The method of any one of paragraphs [42] to [56], wherein the propellant component comprises from 80.0 to 99.99 weight %, preferably from 90.0 to 99.99 weight %, more preferably from 96.5 to 99.99 weight % and especially from 97.5 to 99.95 weight % of the total weight of the pharmaceutical composition.
[58] The method of any one of paragraphs [42] to [57], wherein at least 90 weight %, preferably at least 95 weight % and more preferably at least 99 weight % of the propellant component is 1,1-difluoroethane (HFA-152a).
[59] The method of any one of paragraphs [42] to [57], wherein the propellant component is entirely 1,1-difluoroethane (HFA-152a).
[60] The method of paragraph [58] or [59], wherein the propellant component contains from 0.5 to 10 ppm, e.g. from 1 to 5 ppm, of unsaturated impurities.
[61] The method of any one of paragraphs [42] to [60], wherein at least 95 weight %, preferably at least 98 weight % and more preferably at least 99 weight % of the pharmaceutical composition consists of the drug component, the propellant component, glycerol and ethanol.
[62] The method of any one of paragraphs [42] to [61], wherein the pharmaceutical composition is surfactant-free.
[63] The method of any one of paragraphs [42] to [62], wherein the pharmaceutical composition is free of acid stabilisers.
[64] The method of any one of paragraphs [42] to [63], wherein the pharmaceutical composition consists entirely of the drug component, the propellant component, glycerol and ethanol.
[65] The method of any one of paragraphs [42] to [64], wherein the pharmaceutical composition after storage in uncoated aluminium containers at 40°C and 75 % relative humidity for 1 month produces less than 2.0 % by weight, preferably less than 1.5 % by weight and more preferably less than 1.0 % by weight of impurities from the degradation of the at least one beclomethasone compound based on the total weight of the at least one beclomethasone compound and the impurities for amounts of ethanol up to 15 weight % based on the total weight of the pharmaceutical composition.
[66] The method of any one of paragraphs [42] to [65], wherein the pharmaceutical composition after storage in uncoated aluminium containers at 40°C and 75 % relative humidity for 3 months produces less than 2.5 % by weight, preferably less than 2.0 % by weight and more preferably less than 1.5 % by weight of impurities from the degradation of the at least one beclomethasone compound based on the total weight of the at least one beclomethasone compound and the impurities for amounts of ethanol up to 15 weight % based on the total weight of the pharmaceutical composition.
[67] The method of any one of paragraphs [42] to [66], wherein the pharmaceutical composition is in the form of a suspension.
[68] The method of any one of paragraphs [42] to [66], wherein the pharmaceutical composition is in the form of a solution.
[69] The method of any one of paragraphs [42] to [68], wherein the pharmaceutical composition is free of perforated microstructures.
[70] The method of any one of paragraphs [42] to [69], wherein the pharmaceutical composition is free of pharmaceutically acceptable salts of both cromoglycic acid and nedocromil.
[71] The method of any one of paragraphs [42] to [70], wherein the pharmaceutical composition is free of polymers having amide and/or carboxylic acid ester repeating structural units.
[72] The method of any one of paragraphs [42] to [71], wherein the propellant component has a global warming potential (GWP) of less than 250, preferably less than 200 and more preferably less than 150.
[73] A method of reducing the global warming potential (GWP) of a pharmaceutical composition comprising: (i) a drug component comprising at least one beclomethasone compound selected from beclomethasone and the pharmaceutically acceptable derivatives thereof, particularly beclomethasone dipropionate (BDP), and at least one long acting beta-2-agonist, particularly formoterol fumarate dihydrate; (ii) a propellant component and (iii) glycerol, said method comprising using a propellant component comprising 1,1-difluoroethane (HFA-152a).
[74] The method of paragraph [73], wherein at least 90 weight %, preferably at least 95 weight % and more preferably at least 99 weight % of the propellant component used is 1,1-difluoroethane (HFA-152a).
[75] The method of paragraph [73], wherein the propellant component used is entirely 1,1-difluoroethane (HFA-152a).
[76] The method of paragraph [73], wherein the pharmaceutical composition is as paragraphed in any one of paragraphs 1 to 33.
[77] The method of any one of paragraphs [73] to [75], wherein the propellant component used has a global warming potential (GWP) of less than 250, preferably less than 200 and more preferably less than 150.
[78] The pharmaceutical composition of any one of paragraphs [1] to [33] which is adapted to deliver the compounds making up the drug component in approximately the same proportions that they occur in the pharmaceutical composition.

The present invention is now illustrated but not limited by the following examples.

### Example 1

A number of experiments were conducted to investigate the *in vitro* aerosolization performance of combination drug formulations of beclomethasone dipropionate and formoterol fumarate dihydrate delivered from a metered dose inhaler (MDI) using either HFA-134a or HFA-152a as the propellant.

Pharmaceutical formulations of beclomethasone dipropionate and formoterol fumarate dihydrate were prepared in either HFA-134a or HFA-152a (Mexichem, UK). The drugs were weighed directly into standard uncoated 14 ml aluminium canisters (C128, Presspart, Blackburn, UK) and 10 weight % of anhydrous ethanol (based on the total weight of the formulation) was then added to fully solubilise the drugs. The canisters were then crimped with a 50 µL valve (Bespak, Kings Lynn, UK) following which the propellant was filled into the canisters through the valve using a manual Pamasol crimper/filler (Pamasol, Switzerland). The nominal dose of beclomethasone dipropionate was 250µg and the nominal dose of formoterol fumarate dihydrate was 6µg.

The *in vitro* aerosolization performance of the formulations following storage at ambient conditions for 1 month was studied using a Next Generation Impactor (NGI, Copley Scientific, Nottingham UK) connected to a vacuum pump (GE Motors, NJ, USA). Prior to testing, the cups of the NGI system were coated with 1 % v/v silicone oil in hexane to eliminate particle bounce. For each experiment, three actuations of the valve were discharged into the NGI at 30 L.min⁻¹ as per pharmacopeia guidelines. Following aerosolization, the NGI apparatus was dismantled and the actuator and each part of the NGI was washed down into known volumes of the HPLC mobile phase (see below). The mass of drug deposited on each part of the NGI was determined by HPLC (see below). This protocol was repeated three times for each canister, following which, the fine particle dose (FPD) and fine particle fraction of the emitted dose (FPF_{ED}) were determined.

High performance liquid chromatography (HPLC) was used to determine drug content following the aerosolization studies. A 50 mm x 3 mm Accucore C₁₈ column with a 2.6 µm particle size was used for the analysis. The column was coupled to a UV detector operating at wavelengths of 212 nm and 240 nm depending on which drug was being analyzed. The autosampler was operated at ambient temperature and 100 µl samples were injected into the column for the analyses. The chromatographic conditions are shown in Table 1 below.

**Table 1**

| **Drug** | **Pump Flow Rate (ml.min⁻¹)** | **Mobile Phase (gradient elution)** | **UV Wavelength (nm)** | **Column Temperature (°C)** |
|---|---|---|---|---|
| Beclomethasone Dipropionate and Formoterol Fumarate Dihydrate | 1.0 | Mobile Phase A: 10 mM Ammonium Dihydrogen Orthophosphate at pH 3.0 | 212 and 240 | 40 |
| | | Mobile Phase B: Methanol and Acetonitrile (45:55 v/v) | | |

The composition of the mobile phase was varied as shown in Table 2 below.

**Table 2**

| **Time (mins)** | **Percentage of Mobile Phase A (v/v)** | **Percentage of Mobile Phase B (v/v)** |
|---|---|---|
| 0 | 90 | 10 |
| 2.4 | 0 | 100 |
| 2.7 | 0 | 100 |
| 2.8 | 90 | 10 |
| 4.0 | 90 | 10 |

The results are shown in Tables 3 and 4 below.

**Table 3. In vitro aerosolization performance of combination drug formulations of beclomethasone dipropionate and formoterol fumarate dihydrate in HFA-134a and ethanol as characterised by the fine particle dose, fine particle fraction of the emitted dose (FPF_{ED} (%)), mass median aerodynamic diameter (MMAD) and geometric standard deviation (GSD).**

| | Beclomethasone Dipropionate | Formoterol Fumarate Dihydrate |
|---|---|---|
| Fine Particle Dose (µg) | 98.92 | 3.67 |
| FPF_{ED} % | 63.16 | 66.35 |
| MMAD ± GSD(µm) | 1.41 ± 1.95 | 1.36 ± 2.00 |

**Table 4. In vitro aerosolization performance of combination drug formulations of beclomethasone dipropionate and formoterol fumarate dihydrate in HFA-152a and ethanol as characterised by the fine particle dose, fine particle fraction of the emitted dose (FPF_{ED} (%)), mass median aerodynamic diameter (MMAD) and geometric standard deviation (GSD).**

| | Beclomethasone Dipropionate | Formoterol Fumarate Dihydrate |
|---|---|---|
| Fine Particle Dose (µg) | 105.11 | 3.57 |
| FPF_{ED} % | 55.67 | 60.01 |
| MMAD ± GSD(µm) | 1.57 ± 1.98 | 1.51 ± 2.08 |

### Example 2

A number of experiments were conducted to investigate the effects of glycerol on the *in vitro* aerosolization performance of drug formulations of beclomethasone dipropionate delivered from a metered dose inhaler (MDI) using HFA-152a as the propellant. The aerosolization performance of the combination drug formulations was investigated after initial preparation and after storing under stress storage conditions.

Pharmaceutical formulations of beclomethasone dipropionate were prepared in HFA-152a (Mexichem, UK). The drugs were weighed directly into standard uncoated 14 ml aluminium canisters (C128, Presspart, Blackburn, UK). Anhydrous ethanol in an amount of 5, 10 or 15 weight % based on the total weight of the formulation and glycerol in an amount of 0, 1 or 2 weight %, again based on the total weight of the formulation, were then added to the canisters. The canisters were subsequently crimped with a 50 µL valve (Bespak, Kings Lynn, UK) following which the propellant was filled into the canisters through the valve using a manual Pamasol crimper/filler (Pamasol, Switzerland). The nominal dose of beclomethasone dipropionate was 250µg.

The *in vitro* aerosolization performance of the formulations was tested immediately after preparation with a Next Generation Impactor using the method described in Example 1 above. The results are shown in Table 5 below.

**Table 5. In vitro aerosolization performance of formulations of beclomethasone dipropionate in HFA-152a with varying amounts of ethanol and glycerol as characterised by the fine particle dose (FPM), fine particle fraction of the emitted dose (FPF_{ED} (%)), mass median aerodynamic diameter (MMAD) and geometric standard deviation (GSD).**

| **Wt. % Ethanol** | **Wt. % Glycerol** | **MMAD(µm)±GSD** | **FPM(µg)** | **FPF_{ED} (%)** |
|---|---|---|---|---|
| 5 | 0 | 1.27 ± 1.80 | 114.36 | 65.17 |
| | 1 | 1.55 ± 1.93 | 123.94 | 67.97 |
| | 2 | 1.62 ± 2.06 | 120.25 | 65.69 |
| 10 | 0 | 1.33 ± 1.84 | 117.15 | 59.47 |
| | 1 | 2.09 ± 1.92 | 116.83 | 58.64 |
| | 2 | 2.19 ± 2.12 | 104.23 | 55.76 |
| 15 | 0 | 1.42 ± 2.02 | 102.86 | 50.99 |
| | 1 | 2.29 ± 2.06 | 96.11 | 48.85 |
| | 2 | 2.59 ± 2.12 | 83.04 | 43.57 |

Addition of glycerol acts to increase the MMAD of the formulation thus allowing the deposition of the drug in the various portions of the lung to be optimised.

### Example 3

The stability of combination drug formulations of beclomethasone dipropionate and formoterol fumarate dihydrate in HFA-134a and HFA-152a propellant was investigated at time zero (T=0) and after storage, valve down, for 1 month (T=1M) and 3 months (T=3M) at 40°C and 75% relative humidity (RH) and at 25°C and 60% relative humidity (RH) in uncoated aluminium cans.

The drug formulations were prepared as described in Example 1 above and analysed using the HPLC technique described in Example 1 above.

The results of investigating the chemical stability of the combination drug formulations of beclomethasone dipropionate and formoterol fumarate dihydrate in HFA-152a and HFA-134a in uncoated aluminium cans are shown, respectively, in Tables 6 to 9 below.

**Table 6. Chemical stability of beclomethasone dipropionate in HFA-134a and 10 weight % ethanol in uncoated aluminium cans based on percentage assay and total impurities at T = 0, after storage for 1 month (T = 1M) @ 40°C/75 % RH and 25°C/60 % RH and after storage for 3 months (T = 3M) @ 40°C/75 % RH and 25°C/60 % RH.**

| Time | % Assay (LC) | % total impurities |
|---|---|---|
| Initial time T = 0 | 98.9 | 0.16 |
| T = 1M @ 25/60 | 98.5 | 0.22 |
| T = 1M @ 40/75 | 98.2 | 0.38 |
| T = 3M @ 25/60 | 98.1 | 0.41 |
| T = 3M @ 40/75 | 97.2 | 0.82 |

**Table 7. Chemical stability of beclomethasone dipropionate in HFA-152a and 10 weight % ethanol in uncoated aluminium cans based on percentage assay and total impurities at T = 0, after storage for 1 month (T = 1M) @ 40°C/75 % RH and 25°C/60 % RH and after storage for 3 months (T = 3M) @ 40°C/75 % RH and 25°C/60 % RH.**

| Time | % Assay (LC) | % total impurities |
|---|---|---|
| Initial time T = 0 | 99.9 | <LoQ |
| T = 1M @ 25/60 | 99.5 | 0.09 |
| T = 1M @ 40/75 | 99.8 | 0.08 |
| T = 3M @ 25/60 | 99.5 | 0.08 |
| T = 3M @ 40/75 | 98.9 | 0.12 |

**Table 8. Chemical stability of formoterol fumarate dihydrate in HFA-134a and 10 weight % ethanol in uncoated aluminium cans based on percentage assay and total impurities at T = 0, after storage for 1 month (T = 1M) @ 40°C/75 % RH and 25°C/60 % RH and after storage for 3 months (T = 3M) @ 40°C/75 % RH and 25°C/60 % RH.**

| Time | % Assay (LC) | % total impurities |
|---|---|---|
| Initial time T = 0 | 99.9 | 0.07 |
| T = 1M @ 25/60 | 99.7 | 0.08 |
| T = 1M @ 40/75 | 99.2 | 0.11 |
| T = 3M @ 25/60 | 98.5 | 0.18 |
| T = 3M @ 40/75 | 97.9 | 0.23 |

**Table 9. Chemical stability of formoterol fumarate dihydrate in HFA-152a and 10 weight % ethanol in uncoated aluminium cans based on percentage assay and total impurities at T = 0, after storage for 1 month (T = 1M) @ 40°C/75 % RH and 25°C/60 % RH and after storage for 3 months (T = 3M) @ 40°C/75 % RH and 25°C/60 % RH.**

| Time | % Assay (LC) | % total impurities |
|---|---|---|
| Initial time T = 0 | 99.9 | 0.05 |
| T = 1M @ 25/60 | 99.9 | 0.07 |
| T = 1M @ 40/75 | 99.5 | 0.09 |
| T = 3M @ 25/60 | 99.6 | 0.09 |
| T = 3M @ 40/75 | 99.1 | 0.11 |

It can be seen from the data in Tables 6 to 9 above that pharmaceutical formulations of beclomethasone dipropionate and formoterol fumarate dihydrate exhibit superior chemical stability when blended together with HFA-152a as the aerosolization propellant rather than HFA-134a.

The results also suggest that coated or stainless steel cans are not necessary with HFA-152a based formulations in order for the formulations to demonstrate acceptable chemical stability. Similarly, adequate stability can be attained without the addition of mineral or organic acid to the HFA-152a formulations.

### Example 4

A number of experiments were conducted to investigate the stability of beclomethasone dipropionate in HFA-134a and HFA-152a.

Pharmaceutical formulations of beclomethasone dipropionate were prepared in either HFA-134a or HFA-152a (Mexichem, UK). The drugs were weighed directly into standard uncoated 14 ml aluminium canisters (C128, Presspart, Blackburn, UK). Anhydrous ethanol in an amount of 5, 10 or 15 weight % based on the total weight of the formulation and glycerol in an amount of 0, 1 or 2 weight %, again based on the total weight of the formulation, were then added to the canisters. The canisters were subsequently crimped with a 50 µL valve (Bespak, Kings Lynn, UK) following which the propellant was filled into the canisters through the valve using a manual Pamasol crimper/filler (Pamasol, Switzerland). The nominal dose of beclomethasone dipropionate was 250µg.

The stability of the various beclomethasone dipropionate drug formulations was investigated at time zero (T=0) and after storage, valve down, for 1 month (T=1M) and 3 months (T=3M) at 40°C and 75% relative humidity (RH) in uncoated aluminium cans.

The results of investigating the chemical stability of the beclomethasone dipropionate formulations in HFA-152a and HFA-134a in uncoated aluminium cans are shown, respectively, in Tables 10 and 11 below.

**Table 10. Chemical stability of beclomethasone dipropionate in HFA-134a propellant with varying amounts of ethanol and glycerol in uncoated aluminium cans based on total impurities at T = 0 and upon storage for 1 month (T = 1M) @ 40°C/75 % RH and for 3 months (T = 3M) @ 40°C/75 % RH.**

| **Wt. % Ethanol** | **Wt. % Glycerol** | **% Total Imps T=0** | **% Total Imps T=1M @ 40°C/75 % RH** | **% Total Imps T=3M @ 40°C/75 % RH** |
|---|---|---|---|---|
| 5 | 0 | 0.89 | 1.58 | 2.08 |
| | 1 | 0.34 | 1.91 | 2.14 |
| | 2 | 0.55 | 0.78 | 1.18 |
| 10 | 0 | 1.16 | 1.82 | 2.21 |
| | 1 | 1.34 | 2.25 | 2.49 |
| | 2 | 1.49 | 1.97 | 2.35 |
| 15 | 0 | 1.08 | 2.35 | 4.21 |
| | 1 | 2.88 | 3.15 | 3.99 |
| | 2 | 2.95 | 4.22 | 4.89 |

**Table 11. Chemical stability of beclomethasone dipropionate in HFA-152a propellant with varying amounts of ethanol and glycerol in uncoated aluminium cans based on total impurities at T = 0 and upon storage for 1 month (T = 1M) @ 40°C/75 % RH and for 3 months (T = 3M) @ 40°C/75 % RH.**

| **Wt. % Ethanol** | **Wt. % Glycerol** | **% Total Imps T=0** | **% Total Imps T=1M @ 40°C/75 % RH** | **% Total Imps T=3M @ 40°C/75 % RH** |
|---|---|---|---|---|
| 5 | 0 | 0.08 | 0.16 | 0.35 |
| | 1 | 0.11 | 0.15 | 0.28 |
| | 2 | 0.09 | 0.21 | 0.34 |
| 10 | 0 | 0.21 | 0.35 | 0.42 |
| | 1 | 0.34 | 0.36 | 0.55 |
| | 2 | 0.23 | 0.29 | 0.31 |
| 15 | 0 | 0.56 | 0.89 | 1.11 |
| | 1 | 0.66 | 0.79 | 1.56 |
| | 2 | 0.72 | 0.89 | 1.48 |

It is clear from the data in Tables 10 and 11 above that the stability of beclomethasone dipropionate is significantly higher at all investigated levels of ethanol and glycerol when HFA-152a is used as the propellant rather than HFA-134a.

## Claims

1. A method of improving the stability of a pharmaceutical composition comprising a propellant component, a drug component comprising at least one beclomethasone compound selected from beclomethasone and beclomethasone dipropionate, and ethanol, said method comprising using a propellant component comprising 1,1-difluoroethane (HFA-152a).

2. The method of claim 1, further comprising selecting the components and conditions for the preparation of the pharmaceutical composition to maintain the water content of the pharmaceutical composition below 500 ppm, preferably below 100 ppm, more preferably below 50 ppm, still more preferably below 10 ppm and particularly below 5 ppm based on the total weight of the pharmaceutical composition.

3. The method of claim 1 or 2, wherein the at least one beclomethasone compound is beclomethasone dipropionate.

4. The method of any one of claims 1 to 3, wherein the drug component additionally comprises at least one long acting beta-2-agonist (LABA).

5. The method of claim 4, wherein the at least one long acting beta-2-agonist (LABA) is formoterol fumarate dihydrate.

6. The method of any one of the preceding claims, wherein the drug component additionally comprises at least one long acting muscarinic antagonist.

7. The method of claim 6, wherein the at least one long acting muscarinic antagonist is selected from the group consisting of umeclidinium, ipratropium, tiotropium, aclidinium and the pharmaceutically acceptable salts thereof.

8. The method of claim 6, wherein the at least one long acting muscarinic antagonist is a pharmaceutically acceptable salt of glycopyrrolate, especially glycopyrronium bromide.

9. The method of any one of the preceding claims, wherein at least 90 weight %, preferably at least 95 weight % and more preferably at least 99 weight % of the propellant component is 1,1-difluoroethane (HFA-152a).

10. The method of claim 9, wherein the propellant component contains from 0.5 to 10 ppm, e.g. from 1 to 5 ppm, of unsaturated impurities.

11. The method of any one of the preceding claims, wherein the pharmaceutical composition further comprises glycerol.

12. The method of any one of the preceding claims, wherein the pharmaceutical composition is free of one or more of the following: (i) surfactants, (ii) acid stabilisers, (iii) perforated microstructures, (iv) pharmaceutically acceptable salts of both cromoglycic acid and nedocromil, and (v) polymers having amide and/or carboxylic acid ester repeating structural units.

13. The method of any one of the preceding claims, wherein the pharmaceutical composition after storage in uncoated aluminium containers at 40°C and 75 % relative humidity for 1 month produces less than 2.0 % by weight, preferably less than 1.5 % by weight and more preferably less than 1.0 % by weight of impurities from the degradation of the at least one beclomethasone compound based on the total weight of the at least one beclomethasone compound and the impurities for amounts of ethanol up to 15 weight % based on the total weight of the pharmaceutical composition.

14. The method of any one of the preceding claims, wherein the pharmaceutical composition after storage in uncoated aluminium containers at 40°C and 75 % relative humidity for 3 months produces less than 2.5 % by weight, preferably less than 2.0 % by weight and more preferably less than 1.5 % by weight of impurities from the degradation of the at least one beclomethasone compound based on the total weight of the at least one beclomethasone compound and the impurities for amounts of ethanol up to 15 weight % based on the total weight of the pharmaceutical composition.

15. The method of any one of the preceding claims, wherein the pharmaceutical composition is in the form of a suspension.

16. The method of any one of claims 1 to 14, wherein the pharmaceutical composition is in the form of a solution.

17. The method of any one of the preceding claims, wherein the pharmaceutical composition is stabilised compared to a pharmaceutical composition that uses 1,1,1,2-tetrafluoroethane (HFA-134a) or 1,1,1,2,3,3,3-heptafluoropropane (HFA-227ea) as the propellant but which is otherwise identical.
